(19) Europäisches Patentamt
European Patent Office
Office européen des brevets

(11) Publication number: **0 046 635 B1**

(12) **EUROPEAN PATENT SPECIFICATION**

(45) Date of publication of patent specification: **03.07.85**

(51) Int. Cl.$^4$: **C 07 D 233/64**

(21) Application number: **81303378.4**

(22) Date of filing: **23.07.81**

(54) **Process for the preparation of cimetidine.**

(30) Priority: **21.08.80 FI 802649**

(43) Date of publication of application:
**03.03.82 Bulletin 82/09**

(45) Publication of the grant of the patent:
**03.07.85 Bulletin 85/27**

(84) Designated Contracting States:
**AT CH DE FR GB LI LU NL SE**

(58) References cited:
**FR-A-2 386 525**
**US-A-4 093 621**
**US-A-4 112 234**
**US-A-4 215 217**

**CHEMICAL ABSTRACTS, vol. 91, no. 21, 19. November 1979, Columbus, Ohio, USA LABORATORIO ESTEDI S.L. "N-cyano-N'-methyl-N"-(2-((4-methyl-5-imdazolyl)-methylthio)ethyl)-guanidine" page 667, column 1, abstract no. 175354z**

(73) Proprietor: **Orion-yhtymä Oy**
**PL 19**
**SF-00101 Helsinki 10 (FI)**

(72) Inventor: **Kairisalo, Pekka Juhani**
**Uiskotie 25**
**SF-00950 Helsinki 95 (FI)**
Inventor: **Honkanen, Erkki Juhani**
**Vanha Viertotie 1A 13**
**SF-00300 Helsinki 30 (FI)**

(74) Representative: **Warden, John Christopher et al**
**R.G.C. Jenkins & Co. 12-15, Fetter Lane**
**London EC4A 1PL (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 046 635 B1

Courier Press, Leamington Spa, England.

**Description**

The present invention relates to a novel process for the preparation of crystalline cimetidine, i.e. N-cyano-N'-methyl-N"-(2-(((5-methyl-1H-imidazol-4-yl)methyl)thio)ethyl)guanidine (I)

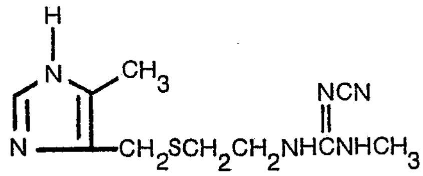

I

wherein N-cyano-N'-methyl-N"-(2-mercaptoethyl)guanidine (II)

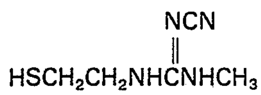

II

is reacted with 4-halomethyl-5-methyl-1H-imidazole (III)

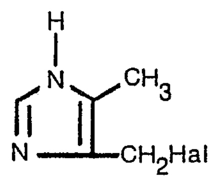

III

wherein the halogen is selected from chlorine or bromine. The process of this invention is characterized in that the reaction is carried out under an inert atmosphere in a medium having a pH range of 8.0 to 9.5 containing water and a water soluble organic solvent.

Cimetidine is an efficient atagonist of the H2-receptors of histamine (Durant et al. *J.Med.Chem.* 20 (1977), 901). It inhibits the secretion of gastric acid induced by histamine and has achieved an important position in the therapy of gastric ulcers.

A process for the preparation of cimetidine is known from the FI—A—79 1116 (examples 1 and 2) wherein 5-methyl-4-(2-aminoethylthiomethyl)imidazole is reacted with N-cyano-N',S-dimethylisothiourea. The purification and the crystallization of the final product, necessary in the process, are, however, difficult to carry out in industrial scale.

Processes wherein N-cyano-N'-methyl-N"-(2-mercaptoethyl)guanidine is reacted with 4-chloromethyl-5-methylimidazole are known from US—A—4,093,621 (example 2), ES—A—458,139 (ref. *Chem. Abstr. 91* (1979), 175 3542) and FR—A—2,386,525. These processes are also very difficult to carry out in industrial scale, because absolutely dry solvents have to be used, sodium alkoxides have to be handled, solvents have to be evaporated, and troublesome purificatons and recrystallizations have to be carried out in them.

It was unexpectedly found out that pure, crystalline cimetidine can be produced in a simple way and in good yield from N-cyano-N'-methyl-N"(2-mercaptoethyl)guanidine and 4-halomethyl-5-methylimidazole when the reaction is carried out under an inert atmosphere in a medium having a pH range of 8.0 to 9.5 containing water and a water soluble organic solvent. Cimetidine is crystallised from the reaction mixture immediately without particular steps.

The reaction is preferred to be carried out at the pH range of 8.5 to 9.0.

In order to increase the yield and the purity of the final product, it is advantageous to perform the reaction at reduced temperature. A suitable temperature is, e.g., 0 to 5°. It is advantageous to use nitrogen as the protective gas.

As for the compounds according to formula (III), 4-chloromethyl-5-methylimidazole and 4-bromomethyl-5-methylimidazole are used. They are quite unstable as free bases, so it is preferable to use them as acid addition salt, such as hydrohalides.

4-Chloromethyl-5-methylimidazole hydrochloride may be prepared, e.g., by the process described in the DE—A—2,800,148, wherein 5-methylimidazole is reacted weith formaldehyde and hydrogen chloride. It is, however, preferable to let 4-hydroxymethyl-5-methylimidazole react with thionyl chloride, whereby 4-chloromethyl-5-methylimidazole hydrochloride is obtained with a quantitative yield.

4-Bromomethyl-5-methylimidazole hydrobromide can be prepared with nearly quantitative yield when 5-methylimidazole is reacted with formaldehyde and hydrobromic acid.

The starting compound according to formula (II) can be prepared from 2-mercaptoethylamine (i.e. cysteamine) and N,S-dimethyl-N'-cyanoisothiourea. When the reaction is carried out in a suitable medium, N-cyano-N'-methyl-N"-(2-mercaptoethyl)guanidine need not be isolated in the intermediate step, but the reaction mixture as such can be used as starting material in the process of this invention.

The process of this invention can be carried out by reacting the starting compounds in a medium containing water and a water soluble organic solvent whose pH has been adjusted to the desired range, e.g., by means of sodium hydroxide. The organic solvent soluble in water, such as alcohol, acetone, or acetonitrile, is used together with water, to ensure that the final product crystallizes easily and in a pure state. A mixture of solvents containing water and methanol in the volume radio of approximately 5:1 is particularly preferable.

According to an embodiment of the process, N-cyano-N′-methyl-N″-(2-mercaptoethyl)guanidine is first fixed in water and an equimolar amount of 4-chloromethyl-5-methylimidazole suspended in methanol is added to the suspension thus obtained. Alternatively, sodium salt of N-cyano-N′-methyl-N″-(2-mercaptoethyl)guanidine can be prepared in the mixture of water and methanol, and 4-chloromethylimidazole hydrochloride dissolved in acidic wate be added to the solution thus obtained.

Particularly pure (over 99%) cimetidine is obtained in a good yield (over 70%) by the process of the invention. If desired, the product is easily obtained even purer by dissolving it as hydrochloride in the mixture of methanol and water, treating it with activated charcoal, and recrystallizing it at the pH range of 8.5 to 9.0. If desired, the product may also be recrystallized from ethoxyethanol, whereby highly pure, crystalline cimetidine is obtained.

The process of this invention has considerable benefits over the known processes for the preparation of cimetidine. The process can be easily applied to the industrial production, because solvents harmful to occupational health, auxiliary materials which are difficult to handle, or high temperatures need not be used in it. The price is also economical, beause of the good yield, low demand of energy, and inexpensive solvents and other auxiliary chemicals.

The following examples illustrate the invention.

Example 1

a) N-cyano-N′-methyl-N″-(2-mercaptoethyl)guanidine

A solution which contains 102 g (0.9 mol) of cysteamine hydrochloride, 700 ml of water, 200 ml of methanol, 116 g (0.9 mol) of N,S-dimethyl-N′-cyanoisothiourea, and 144 g of 50% sodium hydroxide solution. (1.8 mol) is refluxed for 1 hour under nitrogen atmosphere. The solution obtained contains approximately 0.9 mol of the desired compound.

b) N-cyano-N′-methyl-N″-(2-(((5-methylimidazol-4-yl)methyl)thio)ethylguanidine

The pH of the solution obtained in section a) is adjusted to 9.0 by means of concentrated hydrochloric acid and it is cooled, still under nitrogen protection, to −5—0°. 114 g of 50% sodium hydroxide solution (1.8 mol) and a solution containing 150 g (0.9 mol) of 4-chloromethyl-5-methylimidazole hydrochloride, 30 ml of concentrated hydrochloric acid, and 120 ml of water are added simultaneously with vigorous agitation to the solution thus obtained. During the addition the pH of the solution is kept at the range of 8.5 to 9.0 and the temperature below 5°. After the addition the solution is stirred for 20 hours at about 0°. The crystallized product is filtered and washed with cold water and dried. 170 g (75% yield) of the desired product is obtained.

The 4-chloromethyl-5-methylimidazole hydrochloride used as starting material is prepared as follows:

155 g (1.3 mol) of thionyl chloride is added under stirring to a mixture containing 112 g (1 mol) of 4-hydroxymethyl-5-methylimidazole and 1000 ml of dichloromethane. The mixture is refluxed for 2 to 3 hours, cooled and filtered. The filtered product is washed with dichloromethane and dried. 167 g (100%) of the desired product is obtained (mp. 218 to 221°).

Example 2

a) N-cyano-N′-methyl-N″-(2-mercaptoethyl)guanidine

The solution prepared according to section a) of example 1 is made acidic (pH appr. 4) by means of hydrochloric acid and extracted with ethyl acetate. The extract is dried and ethyl acetate is evaporated, whereby the desired product is obtained as colourless oil.

b) N-cyano-N′methyl-N″-(2-(((5-methylimidazol-4-yl)methyl)thio)ethylguanidine

14.2 g (0.09 mol) of the product prepared in section a is mixed with 110 ml of water under nitrogen atmosphere and at 0 to 5°. The pH of the mixture is adjusted to 9 by means of 5N sodium hydroxide solution. 15.0 g (0.09 mol) of 4-chloromethyl-5-methylimidazole hydrochloride suspended in 30 ml of methanol is added during 2 hours. During the addition the temperature is kept at the range of 0 to 5° and the pH at the range of 8.5 to 9.0. The mixture is further stirred for 18 hours at 0°, filtered, the sediment is washed with water and dried. 16.1 g (71%) of cimetidine is obtained.

Example 3

a) 4-bromomethyl-5-methylimidazole hydrobromide

A mixture containing 82 g (1 mol) of 5-methylimidazole, 35 g paraformaldehyde, and 1000 ml of 48% hydrobromic acid is refluxed for 1 hour. The excess of hydrobromic acid is evaporated under reduced pressure, and the residue is crystallized from ethanol. 245 g (96%) of 4-bromomethyl-5-methylimidazole hydrobromide is obtained; mp. 200 to 204°.

b) N-cyano-N′-methyl-N″-(2-(((5-methylimidazol-4-yl)methyl)thio)ethylguanidine

By proceeding as in example 1, but by instead of 4-chloromethyl-5-methylimidazole hydrochloride using 4-bromomethyl-5-methylimidazole hydrobromide, cimetidine is obtained in 65% yield.

**Claims**

1. A process for the preparation of crystalline cimeditine, wherein N-cyano-N′-methyl-N″-(2-mercaptoethyl)-guanidine is reacted with 4-chloro or 4-bromo-methyl-5-methyl-1H-imidazole in a basic medium, characterized in that the reaction is carried out under an inert atmosphere in a medium having a pH range of 8.0 to 9.5 containing water and a water soluble organic solvent.

2. A process as claimed in claim 1, characterized in that the reaction is carried out at the pH range of 8.5 to 9.0.

3. A process as claimed in claim 1 or claim 2, characterized in that reaction is carried out in an atmosphere of nitrogen.

4. A process as claimed in any preceding claim, characterized in that the reaction is carried out at a temperature of 0 to 5°C.

5. A process as claimed in any preceding claim, characterized in that the reaction is carried out by adding an equimolar amount of 4-chloromethyl-5-methyl-1H-imidazole hydrochloride suspended in methanol to the suspension of N-cyano-N′-methyl-N″-(2-mercaptoethyl)guanidine in water during 1 to 3 hours.

6. A process as claimed in any preceding claim, characterized in that the pH of the reaction mixture is adjusted to the desired value by means of sodium hydroxide.

7. A process as claimed in any preceding claim, characterized in that the water soluble organic solvent is selected from alcohol, acetone and acetonitrile.

**Patentansprüche**

1. Ein Verfahren zur Herstellung von kristallinem Cimetidin, wobei N-Cyano-N′-methyl-N″-(2-mercaptoäthyl)-guanidin mit 4-Chlor-oder 4-Brommethyl-5-methyl-1H-imidazol in einem basischen Medium umgesetzt wird, dadurch gekennzeichnet, daß die Reaktion in inerter Atmosphäre in einem Medium mit einem pH-Bereich von 8,0 bis 9,5, das Wasser und ein wasserlösliches, organisches Lösungsmittel enthält, ausgeführt wird.

2. Ein Verfahren nach Anspruch 1, dadurch gekennzeichnet, daß die Reaktion in einem pH-Bereich von 8,5 bis 9,0 ausgeführt wird.

3. Ein Verfahren nach Anspruch 1 oder Anspruch 2, dadurch gekennzeichnet, daß die Reaktion unter Stickstoffatmosphäre ausgeführt wird.

4. Ein Verfahren nach einem vorhergehenden Anspruch, dadurch gekennzeichnet, daß die Reaktion bei einer Temperatur von 0 bis 5°C ausgeführt wird.

5. Ein Verfahren nach einem vorhergehenden Anspruch, dadurch gekennzeichnet, daß die Reaktion ausgeführt wird, indem man eine äquimolare Menge von 4-Chlormethyl-5-methyl-1H-imidazolhydrochlorid, das in Methanol suspendiert ist, während 1 bis 3 Stunden zur Suspension von N-Cyano-N′-methyl-N″-(2-mercaptoäthyl)-guanidin im Wasser zugibt.

6. Ein Verfahren nach einem vorhergehenden Anspruch, dadurch gekennzeichnet, daß der pH-Wert des Reaktionsgemisches mit Natriumhydroxid auf den gewünschten Wert eingestellt wird.

7. Ein Verfahren nach einem vorhergehenden Anspruch, dadurch gekennzeichnet, daß das wasserlösliche, organische Lösungsmittel unter Alkohol, Aceton und Acetonitril ausgewählt ist.

**Revendications**

1. Un procédé pour la préparation de cimétidine cristalline, dans lequel on fait réagir la N-cyano-N′-méthyl-N″-(2-mercaptoéthyl)-guanindine avec la 4-chloro ou la 4-bromo-méthyl-5-méthyl-1H-imidazole en milieu basique, caractérisé en ce que la réaction est effectuée sous atmosphère inerte dans un milieu de pH compris entre 8,0 et 9,5 contenant de l'eau et un solvant organique soluble dans l'eau.

2. Un procédé selon la revendication 1, caractérisé en ce que la réaction est effectuée à un pH compris entre 8,5 et 9,0.

3. Un procédé selon la revendication 1 ou la revendication 2, caractérisé en ce que la réaction est effectuée dans une atmosphère d'azote.

4. Un procédé selon l'une quelconque des revendications précédentes, caractérisé en ce que la réaction est effectuée à une température de 0 à 5°C.

5. Un procédé selon l'une quelconque des revendications précédentes, caractérisé en ce que la réaction est effectuée en ajoutant une quantité équimolaire de chlorhydrate de 4-chlorométhyl-5-méthyl-1H-imidazole en suspension dans le méthanol à la suspension de N-cyano-N'-méthyl-N''-(2-mercapto-éthyl)guanidine dans l'eau pendant 1 à 3 heures.

6. Un procédé selon l'une quelconque des revendications précédentes, caractérisé en ce que le pH du mélange réactionnel est ajusté à la valeur souhaitée au moyen d'hydroxyde de sodium.

7. Un procédé selon l'une quelconque des revendications précédentes, caractérisé en ce que le solvant organique soluble dans l'eau est choisi parmi un alcool, de l'acétone et de l'acétonitrile.